# EUROPEAN PATENT SPECIFICATION

(11) **EP 4 421 062 B1**
(45) Date of publication and mention of the grant of the patent: **03.09.2025**
(21) Application number: 23183704.8
(22) Date of filing: 05.07.2023
(51) Int. Cl.: C07C 1/20, C07C 11/04, C07C 29/151, C07C 31/04, C25B 3/03, C08F 20/44

(54) **PREPARATION OF CHEMICAL PRODUCTS FROM CARBON DIOXIDE WITH REDUCED OR NEGATIVE CARBON DIOXIDE FOOTPRINT**
HERSTELLUNG VON CHEMISCHEN PRODUKTEN AUS KOHLENDIOXID MIT REDUZIERTEM ODER NEGATIVEM KOHLENDIOXIDFUSSABDRUCK
PRÉPARATION DE PRODUITS CHIMIQUES À PARTIR DE DIOXYDE DE CARBONE AVEC EMPREINTE DE DIOXYDE DE CARBONE RÉDUITE OU NÉGATIVE

(30) Priority: 27.02.2023 DE 102023104766
(43) Date of publication of application: 28.08.2024
(73) Proprietor: Obrist Technologies GmbH, 6890 Lustenau (AT)
(72) Inventor: Wesner, Wolfgang, 1220 Wien (AT); Obrist, Frank, 6900 Bregenz (AT)
(74) Representative: Keller Schneider Patentanwaltsgesellschaft mbH

(56) References cited:
- WO-A2-2022/031726
- WO-A2-2022/148837
- US-A1- 2006 235 091
- US-B2- 8 119 091
- BRANC LUIZ ANTÔNIO MELGAÇO NUNES ET AL: "Reinforcement of Concretes with Carbon Fiber Composite", INTERNATIONAL JOURNAL OF COMPOSITE MATERIALS, vol. 4, no. 2, 1 April 2014 (2014-04-01), pages 63 - 68, XP055595049, ISSN: 2166-4919, DOI: 10.5923/j.cmaterials.20140402.04

## Description

### Technical Field

The invention relates to a process, especially a carbon dioxide negative process, for the preparation of a polymeric product. Further, the invention relates to a device, in particular a chemical plant, for carrying out a corresponding process.

### Background Art

As the world continues to face the challenge of climate change, there is growing recognition of the importance of reducing carbon emissions across all sectors of the economy. In response to this challenge, many companies are developing CO₂ neutral chemical products that do not emit any net carbon dioxide into the atmosphere during their production, use, and disposal.

CO₂ neutral chemical products refer to chemicals that are produced using low-carbon or renewable energy sources, and that do not emit any net carbon dioxide during their lifecycle. This means that the carbon emissions associated with their production and use are offset by the removal of an equivalent amount of carbon dioxide from the atmosphere, through activities such as carbon capture and storage or the use of renewable energy sources.

Examples of CO₂ neutral chemical products include bioplastics, which are made from renewable sources such as cornstarch or sugarcane, and can replace traditional, petroleum-based plastics. Other examples include biofuels, which are made from renewable resources such as algae or waste vegetable oil, and can be used as a substitute for fossil fuels in transportation and other applications.

For example, nowadays, polyethylene terephthalate (PET) is mainly produced from the petroleum-derived components terephthalic acid and ethylene glycol. The replacement of terephthalic acid with furandicarboxylic acid is known to be advantageous since one component can be produced from renewable raw materials. The second component mononethylene glycol (MEG) can be produced from bio-ethanol. However, with 100% use of renewable raw material and renewable energy, at best climate neutrality can be achieved.

Attempts are therefore already being made to produce so-called CO₂ negative products. CO₂ negative products refer to products or technologies that actively remove more carbon dioxide from the atmosphere than they emit during their production, use, and disposal. This means that they have a net negative carbon footprint and can help to mitigate the effects of climate change.

In this regard, for example, approaches directed toward the production of carbon negative fuels include those described in US 2010/0311157 A, which teaches the production of biofuels from algae as feedstock. The process is claimed to be carbon negative due to the high absorption of CO₂ by the algae.

WO 2022/031726 A2 describes the air capture of carbon dioxide and its use for the preparation of organic compounds, in particular polymers.

Branco et al. (2014), International Journal of Composite Materials 4(2), 63-68, describes the preparation of concrete reinforced with carbon fibers, which are prepared by pyrolysis of precursor fibers such as polyacrylonitrile.

While the development of CO₂ negative products is an important step towards reducing carbon emissions and mitigating the effects of climate change, there are several challenges associated with their production. One of the main challenges is the cost of implementing these technologies, which can be prohibitively expensive and may require significant investment in research and development.

Another challenge is the scalability of these technologies, as many of the currently known CO₂ negative products are still in the early stages of development and may not be able to be produced at a large scale. In addition, the energy and resource requirements of producing CO₂ negative products may offset the benefits of their carbon negativity, especially if renewable energy sources are not used.

Thus, there is still a need for improved solutions that overcome the aforementioned drawbacks at least partly.

### Summary of the invention

It is therefore the object of the present invention to create an improved process for preparing chemical products, especially polymeric products, with a carbon footprint as low as possible. In particular, the process should allow to produce CO₂ negative chemical products in a cost-effective, scalable and/or efficient manner.

Specifically, the core of the invention is a carbon dioxide negative process, for the preparation of a polymeric product, comprising the steps of:
a) Capturing carbon dioxide (1, 11.1, 11.2) from a gas comprising carbon dioxide by direct air capturing, wherein in a first phase, the gas comprising carbon dioxide is brought into contact with sodium hydroxide to absorb carbon dioxide and to form sodium carbonate; and, in a second phase the sodium carbonate obtained in the first phase is provided in the solid state and at least partially decomposed thermally, whereby gaseous carbon dioxide is released and sodium hydroxide obtained by the thermal decomposition is extracted, and whereby gaseous carbon dioxide and sodium hydroxide are formed out of the sodium carbonate obtained in the first phase and/or out of sodium hydrogencarbonate converted from the sodium carbonate obtained in the first phase at a temperature below 350 °C, preferably below 250 °C, e.g. between 50 and 200 °C, more preferably between 90 °C and 200 °C;
b) Converting at least a part of the captured carbon dioxide (2, 12.1) to an intermediate product comprising an olefin, the olefin comprising propylene; preferably by
   (i) electrochemical reduction (2i, 12.1) of the carbon dioxide in the presence of water; and/or
   (ii) hydrogenation (2ii) of the carbon dioxide with hydrogen to form methanol and subsequently converting the methanol to the olefin, preferably by
      ▪ a methanol-to-olefin reaction (MTO); and/or
      ▪ a methanol-to-gasoline reaction (MTG);
c) Subjecting the propylene of the intermediate product to a derivatization reaction (3, 13.1, 13.2) to obtain acrylonitrile as a propylene derivate, whereby the propylene is subjected to an ammonoxidation reaction to form the acrylonitrile;
d) further subjecting the acrylonitrile to a polymerization reaction (4, 14.1, 14.2) to obtain a polymeric product, whereby acrylonitrile is polymerized to form polyacrylonitrile;

and whereby the polyacrylonitrile is subjected to a pyrolysis reaction to form carbon fibers;
whereby the carbon fibers, in particular in the form of composite material and/or in the form of carbon-fiber-reinforced polymers, are mixed with a processable construction material, in particular a processable mortar or concrete material, to prepare a construction element for a building and/or an infrastructure, for example a base, a support, a beam, a wall, a floor, and/or a ceiling, and wherein at least for step a), in particular for at least steps a) and b), especially for all steps a) - d), energy required for performing the process step(s) is provided in the form of renewable energy

The inventive process is a carbon dioxide negative process. This means in particular that the process is performed such that during the production of the polymeric product, the process removes more carbon dioxide from the environment, e.g. the atmosphere, than it emits during the production of the polymeric product.

However, in principle, the process can also be implemented in a carbon dioxide non-negative manner, e.g. in a carbon dioxide neutral manner or in a manner emitting more carbon dioxide during the production of the polymeric product than the process removes from the environment, especially the atmosphere.

The inventive process provides for an effective, inexpensive, sustainable and robust process for producing polymeric products with a low or even a negative carbon footprint.

Thanks to the inventive combination of process steps, the process can be implemented without any fossil raw materials and fossil energy sources.

Also, the inventive process is highly modular. This means that individual process steps can be effected in one and the same place or plant, respectively, or individual process steps can be performed at different places. For example, steps a) and b) can for example be performed in regions with high solar radiation. The olefins produced in this manner can then be transported to other places, where facilities for performing the further process steps already exist.

The inventive process can be carried out particularly advantageously in the sun belt, along the equator, between the northern and southern turn radius, and/or in deserts. In these locations, almost any amount of solar energy, i.e. solar radiation energy that can be harnessed with photovoltaic cells and/or solar thermal energy that can be made available with solar thermal collectors, is available.

Especially preferred, the energy required for performing the process step(s) in particular is electricity and/or thermal heat. The energy required in the form of electricity is in particular produced by a photovoltaic unit and/or a wind power unit, and/or the energy required in the form of heat is in particular produced with solar thermal collectors. However, other forms of energy can be used as well.

Thus, in a further preferred embodiment, the inventive process comprises a further process step of generating the renewable energy, especially with a photovoltaic unit and/or a wind power unit and/or solar thermal collector.

Especially, the energy required is generated at the same location, especially in the same plant, where step a), especially steps a) and b) or all steps, take(s) place.

The carbon dioxide captured in step a) is at least partly used for producing the polymeric products, and optionally for producing further chemical substances. Additionally, a part of the captured carbon dioxide can be further converted to solid carbon, if desired.

In particular, in step a) the carbon dioxide is captured with a process known as "direct air capture" (DAC).

Direct air capture is a technology that removes carbon dioxide directly from the ambient air using specialized machines. DAC technology typically involves a process where air is passed through a filter and/or solution that selectively captures CO₂, which is then isolated and either stored and/or utilized in other processes.

In the present context, direct air capturing turned out to be especially beneficial since it is cost-effective and the CO₂ can directly be captured from point sources like power plants or industrial facilities where step b) or further process steps take place.

Especially preferred, the capturing of the carbon dioxide in step a) is effected with a chemical absorbent or adsorbent, especially an alkaline substance, for example alkali or alkaline earth hydroxides in solid or liquid form.

According to the invention, in process step a), in a first phase, the gas comprising carbon dioxide is brought into contact with sodium hydroxide, especially a sodium hydroxide solution and/or solid sodium hydroxide, to absorb carbon dioxide and to form sodium carbonate.

Especially, the binding of carbon dioxide from ambient air and/or from exhaust gas streams with slightly elevated carbon dioxide concentrations, can be established by use of low-cost sodium hydroxide, e.g. concentrated aqueous caustic soda, which is readily available on the market and/or can be obtained on site from seawater using the brine of water desalination plants and an electrolysis process.

In the present context, "aqueous caustic soda" or "caustic soda" is meant to be an aqueous sodium hydroxide solution. Especially, the aqueous caustic soda is a concentrated aqueous caustic soda, a saturated aqueous caustic soda or a supersaturated aqueous caustic soda.

Aqueous caustic soda with a sodium hydroxide concentration of at least 10 mol/l is also referred to as concentrated aqueous caustic soda, whereas at room temperature, saturated aqueous caustic soda has a sodium hydroxide concentration of about 32 mol/l and aqueous caustic soda with a higher concentration is called supersaturated aqueous caustic soda. At different temperatures, the saturation concentration changes.

For example, for the first phase in step a), the aqueous caustic soda or the sodium hydroxide solution, respectively, has a sodium hydroxide concentration of 1 - 32 mol/l, in particular 10 - 19 mol/l.

In a second phase of step a), gaseous carbon dioxide and sodium hydroxide, especially a solution of sodium hydroxide, can then be formed out of the sodium carbonate obtained in the first phase and/or out of sodium hydrogencarbonate converted from the sodium carbonate obtained in the first phase. Thereby, the second phase is performed at a temperature below 350 °C, preferably below 250 °C, e.g. between 50 and 200 °C, more preferably between 90 °C and 200°C.

The inventive process in step a) can consume large amounts of energy required at a low temperature level. Heat at a level below 350°C is available from solar thermal energy in practically unlimited quantities and is CO₂ neutral and inexpensive. In this special embodiment described above, the decomposition of the sodium carbonate takes place at low temperatures (below 350 °C), preferably with heat from solar thermal energy and/or waste heat. However, the use of other energy sources, e.g. thermal energy produced via conversion of photovoltaic, wind and/or other energy sources is also possible.

According to the invention, the second phase of step a) is carried out according to the following procedure I:
I. the sodium carbonate obtained in the first phase is provided in the solid state and at least partially decomposed thermally, whereby gaseous carbon dioxide is released and sodium hydroxide obtained by the thermal decomposition is extracted, especially in parallel to the thermal decomposition of the sodium carbonate, preferably by an extraction agent.

In particular, in the inventive method, no calcium compound, especially no calcium hydroxide is used in step a).

Further preferred in the inventive method, no potassium compound, especially no potassium hydroxide is used in step a).

Also, preferably, no microporous hollow fiber membranes are used in step a).

Especially, the sodium carbonate obtained in the first phase of step a) can be temporarily stored, e.g. for at least one minute, at least one hour, at least one day, at least one week or at least one month, before it is used in the second phase. Thereby, the sodium carbonate can for example be stored in solid form and/or in the form of an aqueous solution.

In particular, the first phase is performed such that a water loss, especially a water loss to the atmosphere, is essentially avoided. This can in particular be achieved by sufficiently high amounts or proportions of sodium hydroxide.

In a first preferred embodiment, in the first phase, the gas comprising carbon dioxide is brought into contact with solid sodium hydroxide particles, especially powdery sodium hydroxide.

Thereby, in particular, gas comprising carbon dioxide and moisture, especially residual moisture, is used. Usually, this is for example given when using air or exhaust gas as the gas comprising carbon dioxide in the first phase. This allows for an in-situ generation of aqueous caustic soda that can be reacted with the carbon dioxide in a highly efficient manner. Furthermore, no additional water is required under these conditions.

However, if desired, the gas comprising carbon dioxide can also be brought into contact with solid sodium hydroxide particles, especially powdery sodium hydroxide, in the presence of additional water vapour, especially by humidifying the gas comprising carbon dioxide.

Especially, the solid sodium hydroxide particles are brought into contact with a gas comprising moisture, especially residual moisture, in particular such that aqueous caustic soda, especially concentrated aqueous caustic soda, is formed, and during and/or after formation of the aqueous caustic soda, the obtained caustic soda is brought into contact with the gas comprising carbon dioxide in order to produce the sodium carbonate. The gas comprising moisture can e.g. the gas comprising carbon dioxide to be contacted with the sodium hydroxide and/or another gas comprising moisture.

According to a second highly beneficial embodiment, in the first phase, the gas comprising carbon dioxide is brought into contact with a sodium hydroxide solution, in particular with aqueous caustic soda, to form the sodium carbonate. Especially the aqueous caustic soda is a concentrated aqueous caustic soda, a saturated aqueous caustic soda or a supersaturated aqueous caustic soda.

In particular, the aqueous caustic soda has a sodium hydroxide concentration of 1 - 32 mol/l, in particular 10 - 19 mol/l. This is another possibility to obtain sodium carbonate in a highly efficient manner. Thereby, aqueous caustic soda that is readily available on the market at low prices can directly be used without further treatment.

In the second phase of step a), gaseous carbon dioxide and sodium hydroxide solution, especially an aqueous solution of sodium hydroxide, are formed at a temperature below 350 °C, preferably below 250 °C, more preferably between 90 °C and 200 °C, out of the sodium carbonate obtained in the first phase and/or out of sodium hydrogencarbonate converted from the sodium carbonate obtained in the first phase.

Preferably, the sodium hydroxide, especially the solution of sodium hydroxide, obtained in the second phase is returned to and reused in the first phase of step a). Preferably, before returning the solution of sodium hydroxide, it is concentrated. However, the sodium hydroxide can also be used for other purposes.

Highly preferred, the thermal energy required in the second phase is provided in the form of heat from a solar thermal collector, a solar thermal energy plant and/or waste heat.

Thus, in particular, the inventive method further comprises a step of obtaining heat form a solar thermal collector solar, a solar thermal energy plant and/or waste heat and providing the heat in the second phase of step a) for thermal composition of the sodium carbonate and/or sodium hydrogencarbonate converted from the sodium carbonate obtained in the first phase.

In procedure I of the second phase, the sodium carbonate is provided in the solid state, e.g. by precipitation of the sodium carbonate from the caustic soda in the first phase, and thermally decomposed to release gaseous carbon dioxide. Preferably, the sodium carbonate to be decomposed is present in the form of a particulate material, e.g. a powdery material.

In procedure I, sodium hydroxide (NaOH) produced during the thermal decomposition process of sodium carbonate (Na₂CO₃) is extracted. The extraction further drives the decomposition process so that complete decomposition is possible.

In a preferred embodiment of procedure I, water is used as extraction agent, whereby, preferably, sodium carbonate at least partially precipitates and sodium hydroxide remains in solution. In another preferred embodiment, an aqueous solution of a water soluble alcohol, preferably a water soluble primary alcohol, more preferably methanol, ethanol, n-propanol, n-butanol or a mixture thereof, are used as extraction agent, and, preferably, sodium carbonate at least partially precipitates and sodium hydroxide remains in solution.

In particular, during and/or after the extraction, the extraction agent with sodium hydroxide dissolved therein is subjected to a separation process, especially distillation, in order to recover sodium hydroxide and the extraction agent.

Summarizing, in the procedure I of the second phase for the production of sodium hydroxide solution and release of carbon dioxide from sodium carbonate, the release of carbon dioxide from solid sodium carbonate is preferably effected by direct thermal decomposition, whereby the end product sodium hydroxide solution being preferably removed by extraction to allow further decomposition.

In step b) at least a part of the captured carbon dioxide is converted to an intermediate product comprising an olefin.

Optionally, the so obtained intermediate product can be subjected to a separation step for separating the olefin and/or other substances, e.g. by products, comprised in the obtained intermediate product. Suitable separation processes, such as for example, (fractional) distillation, filtration, membrane separation processes, precipitation, chromatography, extraction, stripping, vapour-liquid separation and the like, are known per se to the skilled person.

In a preferred embodiment, step b) is performed according to option (i) by electrochemical reduction of the carbon dioxide in the presence of water such that a mixture comprising ethylene is obtained.

In particular, the electrochemical reduction is performed with renewable energy, especially with electrical energy generated by a photovoltaic unit and/or a wind power unit.

In particular, the mixture comprising ethylene further comprises at least one compound selected from oxygen, formic acid, formate, carbon monoxide, hydrogen, methane and/or lower alcohols. In particular, the formate is sodium formate. During electrochemical reduction of carbon dioxide, usually these by-products are produced. The proportions of the by-products can be reduced when using special reaction conditions. However, with the present process, this is not a requirement what allows to run the electrochemical reduction in a highly efficient manner.

The climate active methane produced as by-product can further be converted by decomposing it to carbon (C) by recovery of hydrogen gas (H₂), to produce carbon that can be stored for a long time. This results in an overall process in which the ethylene component is not only produced in a CO₂ neutral manner, but an overcompensation of the the CO₂ takes place.

Thus, in a further preferred embodiment, methane contained in the mixture comprising ethylene is decomposed to carbon and hydrogen.

Likewise, the formic acid and/or formate preferably is decomposed to carbon monoxide and hydrogen, especially by pyrolysis.

Especially preferred, the hydrogen and the carbon monoxide are reacted to methanol, the carbon monoxide originating directly from the mixture comprising ethylene and/or from the decomposition of formic acid and/or formate and/or wherein the hydrogen originates directly from the mixture comprising ethylene and/or from the decomposition of methane.

The reaction of hydrogen and carbon monoxide is in particular performed with a catalyst, especially a Cu-based catalyst, in particular a Cu/ZnO/Al₂O₃ catalyst.

Thus, the formic acid, formate, hydrogen and carbon monoxide can be converted to CO₂ negative methanol. The so obtained methanol can be used as a raw material and/or it can be converted to an olefin as described for example in step b) (ii). In this manner, all of the ethylene as well as all of the by-products in the mixture comprising ethylene can be recycled, converted to less problematic substances and/or used for further applications.

The reactions and conditions required for performing the above mentioned reactions are per se known to the person skilled in the art.

According to a further preferred embodiment, step b) is performed according to option (ii). In particular, the hydrogenation of the carbon dioxide and the subsequent conversion to the olefin is performed with renewable energy, especially with energy generated by a photovoltaic unit, a wind power unit, and/or a solar thermal collector.

Thereby, preferably, the carbon dioxide is subjected to a catalytic hydrogenation reaction, whereby, preferably, a Cu-based catalyst and/or an In₂O₃-based catalyst, especially Cu/ZnO/Al₂O₃, is used. Especially, the reaction temperature is chosen from 150-350°C and/or a pressure during the reaction is from of 0.1 - 10 MPa.

In particular, step b) is performed according to option (ii) and the methanol obtained is converted to the olefin, especially ethylene and/or propylene, in a methanol-to-olefin reaction (MTO) with a zeolite catalyst, especially a silicon aluminium phosphate zeolite catalyst. Especially preferred reaction temperatures are in the range of 250- 550°C.

The methanol-to-olefin reaction is a petrochemical process. The process and suitable reaction conditions are known per se in petrochemistry. Thereby, in particular, on a silicon aluminium phosphate zeolite catalyst, a mixture of ethylene and propylene is formed from methanol via the intermediate dimethyl ether. The ratio of propylene (C3) to ethylene (C2) olefins can be varied by selecting the process conditions and ranges for example from 0.77 for the ethylene mode to 1.33 for the propylene mode.

In another preferred embodiment, step b) is performed according to option (ii) and the methanol obtained is converted to the olefin, especially ethylene and/or propylene, in a methanol-to-gasoline reaction (MTG) with an aluminosilicate catalyst, e.g. ZSM-5.

If step b) is performed according to option (ii), optionally after the methanol-to-gasoline (MTG) reaction, steam cracking can be performed. Thereby, in particular, steam cracking is performed with a silico-alumino-phosphate molecular sieve catalyst.

The methanol-to-gasoline reaction is a petrochemical process. The process as such and suitable reaction conditions are known per se in petrochemistry. The methanol-to-gasoline reaction is also known as Mobil process. Typically, the reaction temperature is around 400 °C.

Steam cracking is a petrochemical process in which saturated hydrocarbons are broken down into smaller, often unsaturated, hydrocarbons. It is a robust method for producing olefins such as ethylene and/or propylene.

In step c) an olefin derivate is produced from the propylene of the intermediate product. The derivate is acrylonitrile.

Derivatization is a technique used in chemistry, which converts a chemical compound into a product (the reaction's derivate) of similar chemical structure, called a derivative. Generally, a specific functional group of the compound to be derivatized participates in the derivatization reaction and transforms the chemical compound to a derivate of deviating reactivity, solubility, boiling point, melting point, aggregate state, and/or chemical composition.

Derivatization reactions for obtaining the derivate mentioned above are known to the skilled person.

Especially preferred in step c), ethylene additionally obtained in step b) is reacted with oxygen to obtain ethylene oxide. The so obtained ethylene oxide can then be reacted, in particular hydrolysed, to monoethylene glycol (MEG). The so obtained MEG can be CO₂ negative. Especially, MEG can be used as raw material for the production of further chemical products.

In step c) propylene obtained in step b) is subjected to an ammonoxidation reaction to form acrylonitrile. The so obtained nitrile can be CO₂ negative as well.

Thus, the inventive method allows for obtaining widely used chemical raw materials from carbon dioxide, such as olefins, especially ethylene and/or propylene, as well as methanol and glycols.

According to a highly preferred embodiment, in step d) the obtained glycol, especially monoethylene glycol, is polymerized with terephthalic acid or 2,5-furandicarboxylic acid to obtain polyethylene terephthalate (PET) or polyethylene furanoate (PEF) as the polymeric product. Suitable polymerization initiators, chain transfer agents and polymerization conditions are known to the person skilled in the art.

Thereby the 2,5-furandicarboxylic acid in particular is obtained from biomass, preferably from fructose, especially via the intermediate compound 5-hydroxmethylfurfural.

Likewise, substances and/or monomers used in the inventive process, especially in steps c) and/or step d), preferably are substances and/or monomers that are at least partly, especially fully, biobased.

Compounds from biomass have a measurable proportion of ¹⁴C isotops. By the determination of the ¹⁴C content, it is possible to establish unambiguously whether and in what proportion a compound, e.g. the 2,5-furandicarboxylic acid, is biobased. Thus, biobased compounds differ from non-biobased compounds by a measurable proportion of the ¹⁴C carbon isotope. Compounds which have been produced from fossil raw materials are not biobased and do not have a measurable ¹⁴C content. The ¹⁴C content and the biobased content of raw materials can be determined in accordance with ASTM D6866 "Standard Test Methods for Determining the Biobased Content of Solid, Liquid, and Gaseous Samples Using Radiocarbon Analysis".

When choosing suitable conditions, more CO₂ is removed from the atmosphere during production of the raw materials in step a) - c) than is released during combustion after the polymeric product's lifetime. This fact in turn is transferred to the polymeric products (e.g. PET or PEF) and consumer products made thereof, such as e.g. bottles. Therefore, these polymeric products can be produced to be CO₂ negative.

In another preferred embodiment, in step d) the olefin and/or the glycol is converted to polyethylene glycol (PEG), polypropylene (PP), and/or polyethylene (PE). Suitable polymerization initiators, chain transfer agents and polymerization conditions are known to the person skilled in the art. As explained above for PEG and PEF, these polymeric products can be produced to be CO₂ negative as well.
in step c) acrylonitrile is produced, especially in a Sohio process, which then is polymerized in step d) to form polyacrylonitrile. Likewise, this polymer can be produced to be CO₂ negative as well.

Furthermore, the polyacrylonitrile is subjected to a pyrolysis reaction to form carbon fibers. In particular, the carbon fibers can be combined with other materials, e.g. with polymeric products obtained with the inventive process, to form a composite material or a carbon-fiber-reinforced polymer.

Carbon fibers are widely used for application in aerospace industry, automotive industry and/or civil engineering. Being able to produce carbon fibers, composite materials or carbon-fiber-reinforced polymers being CO₂ negative, is highly beneficial since the carbon footprint of products comprises the carbon fibers can be lowered.

The carbon fibers, in particular in the form of composite materials or carbon-fiber-reinforced polymers, are mixed with a processable construction material, in particular with a processable mortar or concrete material, to prepare a construction element for a building and/or an infrastructure. The construction element is for example a base, a support, a beam, a wall, a floor, and/or a ceiling.

The processable construction material in particular comprises a binder, aggregates and water. The binder can be selected from cements and/or other binder materials, such as e.g. geopolymers.

In particular, the processable construction material comprises a geopolymer binder, in particular as the only binder. With regard to CO₂ balance, such construction materials are highly beneficial. Furthermore, since with such kind of binders metallic reinforcements are probelamtic due to the rather low pH, the use of carbon fibers as reinforcement element is highly beneficial. The combination of geopolymer binders and carbon fibers produced according to the inventive process is highly advantageous with regard to CO₂ balance.

In a further preferred embodiment, the inventive process additionally comprises a step of providing hydrogen and/or oxygen by hydrolysis of water. In particular, the so obtained hydrogen and/or oxygen then is used in step b) (ii) and/or in step c).

In particular, the hydrolysis is performed with renewable energy, especially with electrical energy generated by a photovoltaic unit and/or a wind power unit.

A further aspect is related to a construction element obtainable by the process as described above. Especially, the construction element is for example a base, a support, a beam, a wall, a floor, and/or a ceiling of a building or of an infrastructure.

Another aspect is directed to a device, in particular a chemical plant, for performing the process as described above, comprising:
a) A capturing unit, especially a sorption unit, that is configured for capturing carbon dioxide from a gas comprising carbon dioxide, in particular from air or exhaust gas, especially by direct air capturing, and wherein in a first phase, the gas comprising carbon dioxide can be brought into contact with sodium hydroxide, especially a sodium hydroxide solution and/or solid sodium hydroxide, to absorb carbon dioxide and to form sodium carbonate; and, in a second phase the sodium carbonate obtained in the first phase can be provided in the solid state and at least partially decomposed thermally, whereby gaseous carbon dioxide can be released and sodium hydroxide obtained by the thermal decomposition can be extracted, especially in parallel to the thermal decomposition of the sodium carbonate, preferably by an extraction agent;
b) A conversion unit, that is configured for at least a part of the carbon dioxide captured with the capturing unit to an intermediate product comprising an olefin, especially ethylene and/or propylene; whereby the conversion unit preferably comprises:
   - A reduction unit for electrochemical reduction of the carbon dioxide in the presence of water; and/or
   - A hydrogenation unit for hydrogenation of the carbon dioxide with hydrogen to form methanol and subsequently converting the methanol to the olefin;
c) Optionally, a derivatization unit, which is configured for derivatization at least a part of the olefin of the intermediate product to an olefin derivative;
d) A polymerization unit, for polymerizing the olefin and/or olefin derivative, in particular with at least one further monomer, to obtain a polymeric product.

In another preferred embodiment, the device further comprises at least one unit selected from
- a decomposition unit in which formic acid and/or formate is decomposed to carbon monoxide and hydrogen,
- a decomposition unit in which methane is decomposed to carbon and hydrogen, and
- a synthesis unit in which hydrogen and carbon monoxide are converted to methanol;
- a hydrolysis unit for generating hydrogen and/or oxygen from water.

Optionally, the device further comprises a carbonation unit for producing carbon (C) from the captured carbon dioxide and hydrogen.

Especially, the carbonation can be effected via a Bosch reaction, which is in particular performed in a two-stage reaction with quantitative water removal, preferably in the presence of an iron, cobalt and/or nickel catalyst.

Additionally, the plant preferably comprises an energy unit that is configured for producing renewable energy, especially a photovoltaic unit, a wind power unit and/or a solar thermal unit, for generating the energy required for performing the process step(s). In particular, the energy unit provides energy in the form of electricity and/or thermal heat.

In particular, the device is configured for capturing at least 200'000 tons CO₂ per year, preferably at least 500'000 tons CO₂ per year.

According to a highly preferred embodiment, the device is part of a plant, in particular a chemical plant. However, individual units of the device can be located at different locations. In this case, the chemical plant is a distributed chemical plant.

Another aspect described herein, but not part of the presently claimed invention, is related to a combined power station and chemical plant, for reducing the carbon dioxide content in atmospheric air, in particular for the reduction of the carbon dioxide content in atmospheric air and proportionately in water, preferably sea water, and the production of polymeric products.

Especially, the combined power station and chemical plant comprises:
- at least one electrolysis unit for oxygen production, which is connected to at least one water supply line for receiving a quantity of water (M_{H2O}) and is adapted to supply an absorbed amount of water (M_{H2O}) by electrolysis into an electrolysis into a partial quantity of oxygen (M_{O2}) and a partial quantity of hydrogen fraction;
- at least one conversion unit, especially as described above, that is configured for converting at least a part of the carbon dioxide captured with the carbon dioxide sorption unit to an intermediate product comprising an olefin, especially ethylene and/or propylene; whereby the conversion unit preferably comprises:
   - A reduction unit for electrochemical reduction of the carbon dioxide in the presence of water; and/or
   - A hydrogenation unit for hydrogenation of the carbon dioxide with hydrogen to form methanol and subsequently converting the methanol to the olefin;
- optionally, at least a derivatization unit for derivatization at least a part of the olefin of the intermediate product to an olefin derivative;
- At least a polymerization unit, for polymerizing the olefin and/or olefin derivative, in particular with at least one further monomer, to obtain a polymeric product;
- at least one hydrogen transport device, connecting the electrolysis unit to a carbonation unit for carbon synthesis, and preferably also for connecting the electrolysis unit to the conversion unit, especially to the hydrogenation unit of the conversion unit;
- at least one carbon dioxide sorption unit, especially as described above, which is configured for the purification of ambient air of an external atmosphere surrounding the unit, at least one air inlet for supplying the ambient air and the ambient air and at least one downstream sorbent device which is adapted to extract a quantity of carbon dioxide from the ambient air, especially as described above; and
- at least one carbon dioxide transport means, which connects the carbon dioxide sorption unit with the carbonation unit and with the conversion unit,
- wherein said electrolysis unit having at least one oxygen outlet for discharging the oxygen fraction (M_{O2}) and the carbon dioxide sorption unit has at least one air outlet for discharging purified ambient air, wherein the oxygen outlet and the air outlet opening into the outside atmosphere, the carbonation unit having a carbon outlet for removing carbon for the removal of carbon, and
- wherein at least one power generation unit for the self-sufficient power supply power supply of the plant is provided, which, for power generation one or more, in particular exclusively regenerative energy sources for power generation.

In particular, further features of the plant can be implemented according to DE 10 2021 104 746 B3.

With the above described plant, the units are configured for performing the inventive process. Thereby, in particular, the carbon dioxide (ab)sorption units and the sorber units are connected to a source of solar thermal heat and/or waste heat.

Other advantageous embodiments and combinations of features come out from the detailed description below and the entirety of the claims.

### Brief description of the drawings

The drawings used to explain the embodiments show:
- Fig. 1: a flow diagram of a process for producing a polymeric product;
- Fig. 2: a flow diagram showing an implementation of the first step 1 of the process show in Fig. 1 whereby carbon dioxide is directly captured from a gas comprising carbon dioxide with sodium hydroxide;
- Fig. 3: a sketch of a membrane-capacitive process for the disproportionation of sodium carbonate in the process shown in Fig. 2;
- Fig. 4: a flow diagram showing an implementation of the process of Fig. 1 for producing polyethylene terephthalate and polyethylene furanoate;
- Fig. 5: a flow diagram showing a further implementation of the process of Fig. 4 whereby beside polyethylene terephthalate and polyethylene furanoate further intermediate products are used;
- Fig. 6: a flow diagram showing the production of carbon fibers or carbon-fiber-reinforced polymers.

In the figures, the same components are given the same reference symbols.

### Preferred embodiments

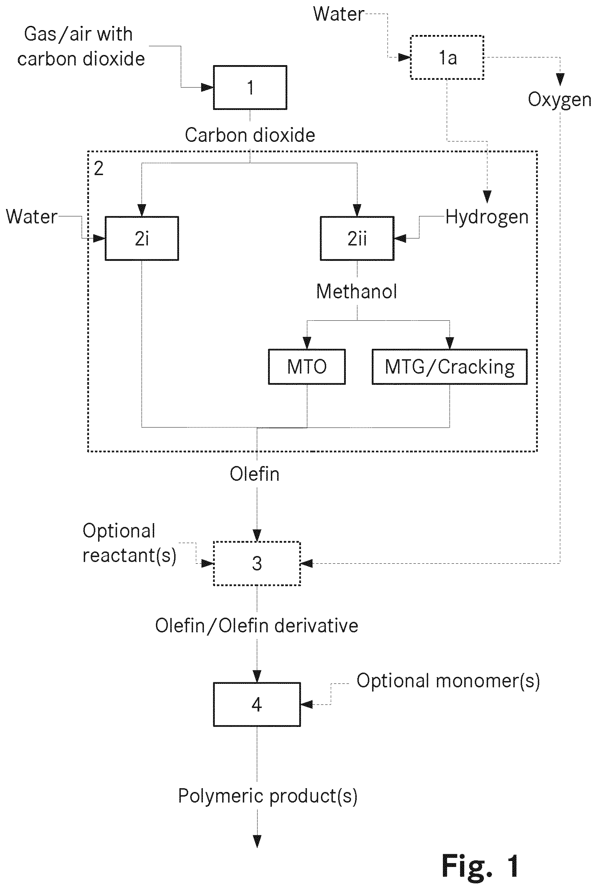

Figure 1 shows a flow diagram of a process for producing a polymeric product. Specifically, in a capturing unit 1 (implementing step a) of the invention), carbon dioxide from a gas comprising carbon dioxide, in particular from air or exhaust gas, is captured. The so obtained carbon dioxide then is subjected to a conversion unit 2(i) and/or 2(ii) (implementing step b) of the invention). In unit 2(i), at least a part of the captured carbon dioxide is subjected to an electrochemical reduction in the presence of water to obtain an intermediate product comprising an olefin, especially ethylene and/or propylene. In unit 2(ii) the carbon dioxide first is hydrogenated with hydrogen to form methanol. Subsequently, the methanol is subjected to a methanol-to-olefin reaction MTO and/or a methanol to gasoline reaction MTG to obtain an intermediate product comprising an olefin, especially ethylene and/or propylene.

In optional derivatization unit 3 (implementing optional step c) of the invention), the olefin can be subjected to a derivatization reaction to obtain an olefin derivate, e.g. a glycol and/or acrylonitrile. Depending on the derivatization reaction, reactants such as oxygen, water and/or ammonia are supplied.

Subsequently, the olefin and/or the olefin derivate, if desired with at least one further monomer, is subjected to a polymerization unit 4 to obtain a polymeric product (implementing step d) of the invention).

Hydrogen used in unit 2(ii) as well as oxygen suitable for the derivatization unit 3, can be produced from water in an optional hydrolysis unit 1a.

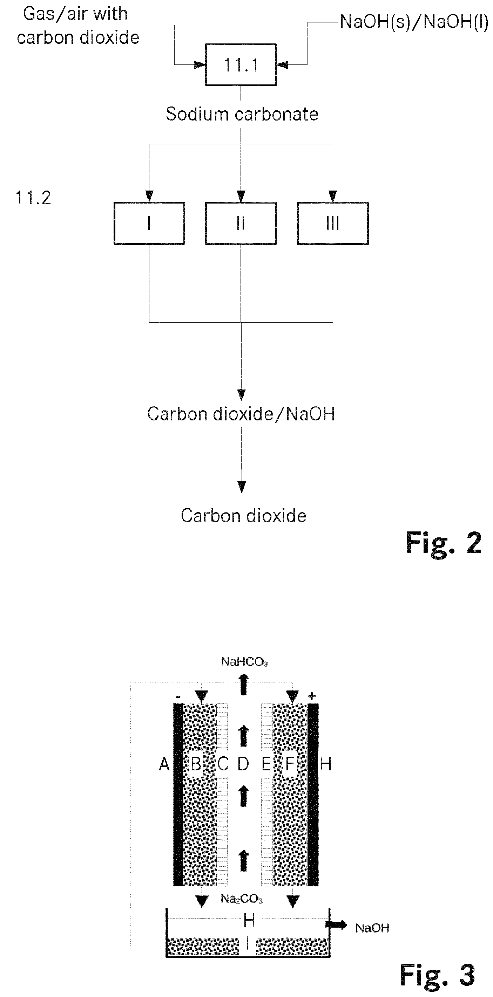

Figure 2 shows a flow diagram showing an implementation of the process in the unit 1 of the process show in Fig. 1 whereby carbon dioxide is directly captured from a gas comprising carbon dioxide with sodium hydroxide.

In the first step 11.1, the gas comprising carbon dioxide (CO₂) is brought into contact with sodium hydroxide (NaOH), e.g. a sodium hydroxide solution and/or solid sodium hydroxide, to absorb the carbon dioxide. During step 11.1, sodium carbonate (Na₂CO₃) is formed. Depending on the specific implementation of step 11.2, the sodium carbonate is provided as a solid (procedure I) or in the form of an aqueous solution (procedures II and III).

In the second step 11.2, the gaseous carbon dioxide and diluted sodium hydroxide solution, especially a solution of sodium hydroxide, are formed at a temperature below 350 °C, preferably below 250 °C, e.g. between 50 and 200 °C, more preferably between 90 °C and 200 °C, out of the obtained sodium carbonate obtained in the first step a) and/or out of sodium hydrogencarbonate converted from the obtained sodium carbonate obtained in the first step a). Thereby, step 11.2 can be carried out according to at least one of the procedures I, II and/or III.

In procedure I, the sodium carbonate obtained in step 11.1 is used in the solid state and at least partially decomposed thermally, e.g. at a temperature of 90 °C and 200 °C, whereby gaseous carbon dioxide is released. The additional sodium hydroxide obtained by the thermal decomposition is extracted, especially parallel to the thermal decomposition of the sodium carbonate, by an extraction agent, e.g. water or an aqueous solution of alcohols.

In procedure II (not part of the claimed invention), the sodium carbonate obtained in step 11.1 is used in the form of an aqueous solution, which is at least partially decomposed thermally by heating the aqueous solution, especially to a temperature below the boiling point of the aqueous solution, such that gaseous carbon dioxide is released from the solution.

In procedure III (not part of the claimed invention), the sodium carbonate obtained in step 11.1 is used in the form of an aqueous solution and at least partially separated by an ion-selective membrane arrangement into sodium hydrogencarbonate and sodium hydroxide, wherein the obtained sodium hydrogencarbonate in a third step is at least partially thermally decomposed into carbon dioxide and sodium carbonate. In Figure 3 an exemplary embodiment of an ion-selective membrane arrangement is shown.

Figure 3 shows a sketch of a membrane-capacitive arrangement and process for the disproportionation of sodium carbonate that can be used in procedure III. Thereby, a concentrated sodium carbonate solution (Na₂CO₃ in water) is introduced into the centre cell D at one end and pumped in countercurrent flow to a slurry of conductive activated carbon particles flowing through a cathode cell B and an anode cell F. The centre cell D is confined by two cation-selective membranes C and E. Sodium ions migrate through the membrane C into the cathode cell B, protons from the anode cell F migrate through the membrane E into the centre cell D. The cathode cell B is confined on the outside by an edge electrode A made of a conductive material such as carbon or stainless steel, which is conductively connected to the negative pole of a current source (not shown). The anode cell F is bounded on the outside by an edge electrode G which is conductively connected to the positive pole of an external current source. The slurries of conductive activated carbon are pumped through the anode and cathode cells B, F in countercurrent flow to the centre cell D.

The charge of the activated carbon particles applied via the edge electrode A is neutralised in the cathode cell B by sodium ions migrating from the centre cell D via the cation-selective membrane C. In the anode cell F, the positive charge of the activated carbon particles applied by the edge electrode G is balanced by hydroxide ions from the dissociation of water. The protons released in this process migrate in the electric field through the cation-selective membrane e into the middle cell D.

After passing through the cathode cell B or the anode cell F, the carbon slurry of the two cells is combined outside the cell, whereby sodium hydroxide is formed in a neutralisation reaction after the electric field is removed. The carbon particles are discharged in the process. The discharged carbon particles I are separated from the caustic soda H by filtration or sedimentation, the carbon particles I are slurried with fresh water and returned to the cells B and F. The caustic soda H is added to the absorption unit in step 11.1 of the process.

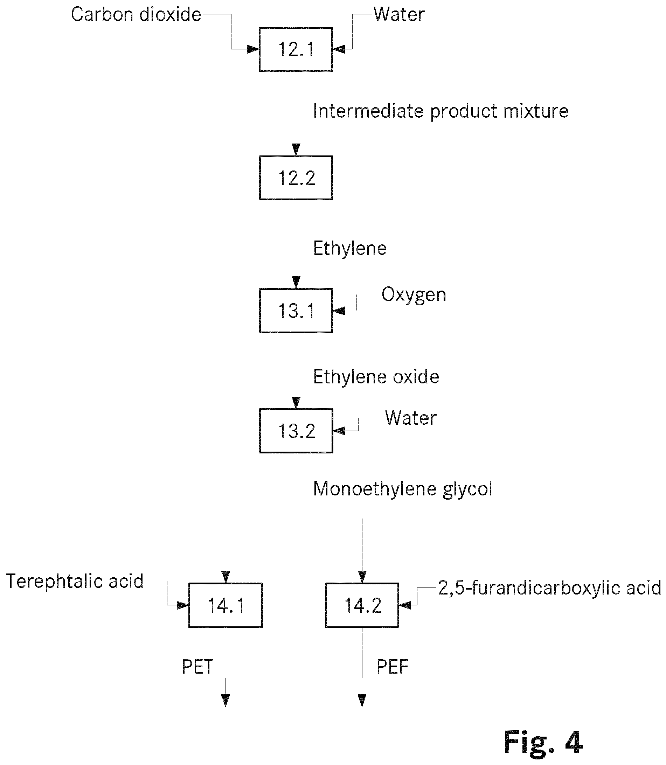

Figure 4 (not part of the claimed invention) shows a flow diagram showing an implementation of the process of Fig. 1 for producing polyethylene terephthalate and polyethylene furanoate.

Carbon dioxide captured as explained with Figure 1 is subjected to an electrochemical reduction step 12.1 in the presence of water to obtain an intermediate product mixture. The product mixture then is subjected to a separation step 12.2 to isolate ethylene comprised in the intermediate product mixture. Steps 12.1 and 12.2 are specific implementations of the conversion step 2(i) shown in Figure 1.

Subsequently, the so obtained ethylene is subjected to an oxidation step 13.1 to produce ethylene oxide, which is treated in a hydrolysis step 13.2 with water to produce monoethylene glycol as an ethylene derivate. Steps 13.1 and 13.2 are specific implementations of the optional derivatization step 3 of Figure 1.

In the following the monoethylene glycol is polymerized together with terephtalic acid to produce polyethylene terephtalat (PET) in polymerization step 14.1 and/or the monoethylene glycol is polymerized together with 2,5-furandicarboxylic acid to produce polyethylene furanoate (PEF) in polymerization step 14.2. Steps 14.1 and 14.2 are specific implementations of the optional polymerization step 4 of Figure 1.

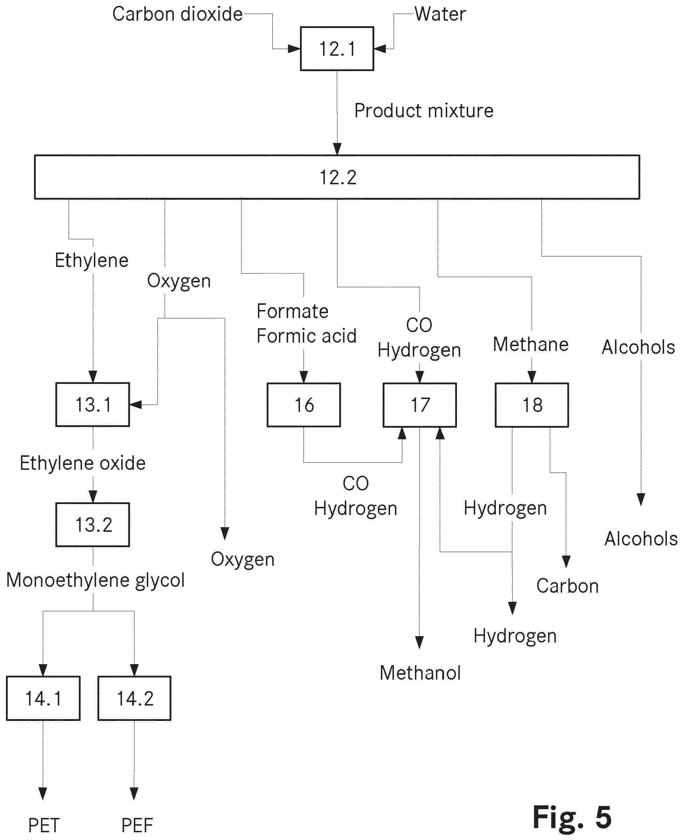

Figure 5 (not part of the claimed invention) shows a flow diagram showing a further implementation of the process of Fig. 4 whereby beside the PET and PEF, the ethylene as well as all of the by-products in the mixture comprising ethylene recycled, converted to less problematic substances or used for further applications.

Specifically, the carbon dioxide captured as explained with Figure 1 is subjected to an electrochemical reduction step 12.1 in the presence of water to obtain an intermediate product mixture. The product mixture then is subjected to a separation step 12.2 to isolate ethylene, oxygen, formate, formic acid, carbon monoxide (CO), hydrogen, methane and alcohols comprised in the intermediate product mixture. Suitable methods for separating and isolating these substances are known to the skilled person .

The separated ethylene is subjected to the same treatment as described with Figure 4 to produce PET and/or PEF.

The oxygen can be used in step 13.1 for the derivatization of the ethylene and/or it can be used for other applications.

Formate, which is present as sodium formate, can be decomposed on heating to > 300°C with elimination of hydrogen and monoxide (1:1) and leaves Na₂CO₃, especially by a pyrolysis step 16. Formic acid can either be decomposed in step 16 into carbon dioxide and hydrogen and/or into carbon monoxide and water, depending on the temperature and type of catalyst used. Both reactions are in equilibrium.

The products of the pyrolysis step 16 and further carbon monoxide and hydrogen separated from the intermediate product mixture can be reacted to methanol in a hydrogenation step 17.

Likewise, methane separated from the intermediate product mixture can be decomposed to hydrogen and carbon (C) in a decomposition step 18. The carbon can be stored and thereby is removed from the atmosphere on a long term. The hydrogen obtained in this process can be reused in the hydrogenation step 17 or I t can be used for other applications.

Alcohols comprised in the intermediate product mixture for example be used in in chemical industry.

With the method shown in Fig. 5, all of the components comprised in the intermediate product mixture can be used as such and/or or converted to other useful substances.

However, the present invention is not restricted to the examples shown in the specific embodiments. For example, instead of PET and PEF, other polymeric substances can be produced, such as e.g. polyethylene, polypropylene, polystyrene, polyacrylnitrile and many more polymeric substances.

Furthermore, the polymeric substances produced with the inventive process can be further converted to other products. For example, polyacrylonitrile can be convertred to carbon fibers, which can be used as reinforcing elements in building materials and/or for industrial applications.

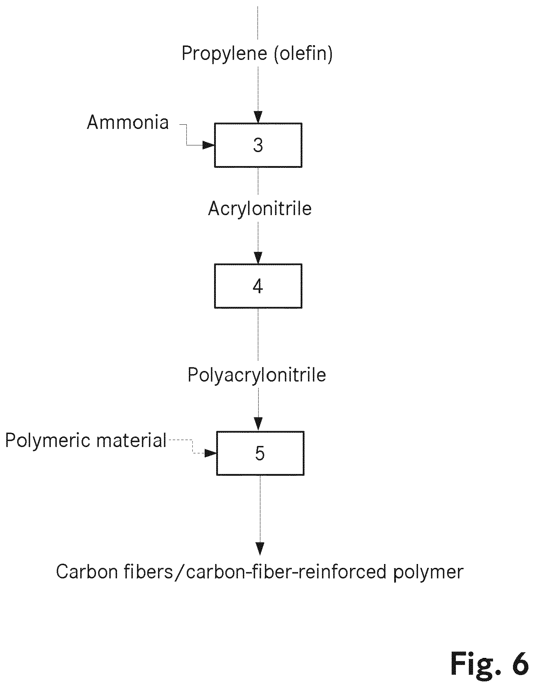

Fig. 6 shows a flow diagram showing the production of carbon fibers or carbon-fiber-reinforced polymers. Thereby, propylene obtained in conversion units 2(i) or 2(ii) (cf. Fig. 1) is converted in the derivatization unit 3 with ammonia to acrylonitrile. Subsequently, the acrylonitrile is subjected to a polymerization reaction in polymerisation unit 4 to form polyacrylonitrile.

In a further step, the polyacrylonitrile is decomposed in a pyrolysis unit 5 to form carbon fibers. Optionally, the carbon fibers can be embedded in a polymeric material (which can be produced with the inventive process as well or otherwise) to form carbon-fiber-reinforced polymers.

The so obtained carbon fibers or carbon-fiber-reinforced polymers can for example be used for applications in aerospace industry, automotive industry and/or civil engineering, e.g. as reinforcement in construction materials, such as for example mortar or concrete structures.

The carbon fibers and carbon-fiber-reinforced polymers can be produced in a CO₂ negative manner. This in turn allows the carbon footprint of products comprising these components to be lowered.

## Claims

1. A carbon dioxide negative process, for the preparation of a polymeric product, comprising the steps of:
a) Capturing carbon dioxide (1, 11.1, 11.2) from a gas comprising carbon dioxide by direct air capturing, wherein in a first phase, the gas comprising carbon dioxide is brought into contact with sodium hydroxide to absorb carbon dioxide and to form sodium carbonate; and, in a second phase the sodium carbonate obtained in the first phase is provided in the solid state and at least partially decomposed thermally, whereby gaseous carbon dioxide is released and sodium hydroxide obtained by the thermal decomposition is extracted, and whereby gaseous carbon dioxide and sodium hydroxide are formed out of the sodium carbonate obtained in the first phase and/or out of sodium hydrogencarbonate converted from the sodium carbonate obtained in the first phase at a temperature below 350 °C, preferably below 250 °C, e.g. between 50 and 200 °C, more preferably between 90 °C and 200 °C;
b) Converting at least a part of the captured carbon dioxide (2, 12.1) to an intermediate product comprising an olefin, the olefin comprising propylene; preferably by
(i) electrochemical reduction (2i, 12.1) of the carbon dioxide in the presence of water; and/or
(ii) hydrogenation (2ii) of the carbon dioxide with hydrogen to form methanol and subsequently converting the methanol to the olefin, preferably by
▪ a methanol-to-olefin reaction (MTO); and/or
▪ a methanol-to-gasoline reaction (MTG);
c) Subjecting the propylene of the intermediate product to a derivatization reaction (3, 13.1, 13.2) to obtain acrylonitrile as a propylene derivate, whereby the propylene is subjected to an ammonoxidation reaction to form the acrylonitrile;
d) further subjecting the acrylonitrile to a polymerization reaction (4, 14.1, 14.2) to obtain a polymeric product, whereby acrylonitrile is polymerized to form polyacrylonitrile;
and whereby the polyacrylonitrile is subjected to a pyrolysis reaction to form carbon fibers;
whereby the carbon fibers, in particular in the form of composite material and/or in the form of carbon-fiber-reinforced polymers, are mixed with a processable construction material, in particular a processable mortar or concrete material, to prepare a construction element for a building and/or an infrastructure, for example a base, a support, a beam, a wall, a floor, and/or a ceiling, and wherein at least for step a), in particular for at least steps a) and b), especially for all steps a) - d), energy required for performing the process step(s) is provided in the form of renewable energy.

2. Process according to claim 1, further comprising a step of generating the renewable energy, especially with a photovoltaic unit and/or a wind power unit and/or solar thermal collector, whereby, in particular, the energy is generated in a plant where step a), especially steps a) and b) or all steps, take(s) place.

3. Process according to any of preceding claims, whereby step b) comprises the step of converting at least a part of the captured carbon dioxide (2, 12.1) to an intermediate product comprising an olefin, the olefin comprising ethylene, whereby step b) is performed according to option (i) such that a mixture comprising ethylene and at least one compound selected from oxygen, formic acid, formate, carbon monoxide, hydrogen, methane and/or lower alcohols is obtained.

4. Process according to claim 3, whereby the formic acid and/or formate is decomposed to carbon monoxide and hydrogen and/or the methane is decomposed to carbon and hydrogen.

5. Process according to claim 3 or 4, whereby the hydrogen and the carbon monoxide are reacted to methanol, the carbon monoxide originating directly from the mixture comprising ethylene and/or from the decomposition of formic acid and/or formate and/or wherein the hydrogen originates directly from the mixture comprising ethylene and/or from the decomposition of methane.

6. Process according to any of preceding claims, whereby step b) is performed according to option (ii) and the carbon dioxide is subjected to a catalytic hydrogenation reaction to obtain the methanol, whereby a Cu-based catalyst and/or an In₂O₃-based catalyst, especially Cu/ZnO/Al₂O₃, is used and/or step b) is performed according to option (ii) and the methanol obtained is converted to the olefin, especially ethylene and/or propylene, in a methanol-to-olefin reaction with a zeolite catalyst, especially a silicon aluminium phosphate zeolite catalyst and/or step b) is performed according to option (ii) and the methanol obtained is converted to the olefin, especially ethylene and/or propylene, in a methanol-to-gasoline reaction with an aluminosilicate catalyst, e.g. ZSM-5.

7. Process according to any of preceding claims, whereby step b) comprises the step of converting at least a part of the captured carbon dioxide (2, 12.1) to an intermediate product comprising an olefin, the olefin comprising ethylene, and subjecting the olefin of the intermediate product to a derivatization reaction (3, 13.1, 13.2) to obtain a glycol, whereby in step d) the obtained glycol, especially monoethylene glycol, is polymerized with terephthalic acid or 2,5-furandicarboxylic acid to obtain polyethylene terephthalate (PET) or polyethylene furanoate (PEF) as the polymeric product, wherein preferably the 2,5-furandicarboxylic acid is obtained from biomass, preferably from fructose, in particular via the intermediate compound 5-hydroxmethylfurfural.

8. Process according to claim 7, whereby in step d) the olefin and/or the glycol is converted to polyethylene glycol (PEG), polypropylene (PP), and/or polyethylene (PE).

9. Process according to any of preceding claims, whereby the processable construction material preferably comprises a geopolymer binder.

10. Device, in particular a chemical plant, for performing the process according to any of claims 1 - 9, comprising:
a) A capturing unit (1), especially a carbon dioxide sorption unit, that is configured for capturing carbon dioxide from a gas comprising carbon dioxide, in particular from air or exhaust gas, especially by direct air capturing, and wherein in a first phase, the gas comprising carbon dioxide can be brought into contact with sodium hydroxide, especially a sodium hydroxide solution and/or solid sodium hydroxide, to absorb carbon dioxide and to form sodium carbonate; and, in a second phase the sodium carbonate obtained in the first phase can be provided in the solid state and at least partially decomposed thermally, whereby gaseous carbon dioxide can be released and sodium hydroxide obtained by the thermal decomposition can be extracted, especially in parallel to the thermal decomposition of the sodium carbonate, preferably by an extraction agent;
b) A conversion unit (2), that is configured for converting at least a part of the carbon dioxide captured with the capturing unit to an intermediate product comprising an olefin, especially ethylene and/or propylene; whereby the conversion unit preferably comprises:
- A reduction unit (2i) for electrochemical reduction of the carbon dioxide in the presence of water; and/or
- A hydrogenation unit (2ii) for hydrogenation of the carbon dioxide with hydrogen to form methanol and subsequently converting the methanol to the olefin;
c) Optionally, a derivatization unit (3) for derivatizing at least a part of the olefin of the intermediate product to an olefin derivative;
d) A polymerization unit (4), for polymerizing the olefin and/or olefin derivative, in particular with at least one further monomer, to obtain a polymeric product.

11. A device according to claim 10 further comprising an energy unit that is configured for producing renewable energy, especially a photovoltaic unit, a solar thermal unit and/or a wind energy unit.

12. A device according to claim 10 or 11, further comprising at least one unit selected from
- a separation unit (12.2) in which by-products, especially at least one compound selected from oxygen, formic acid, formate, carbon monoxide, hydrogen, methane and/or lower alcohols, can separated from the intermediate product comprising the olefin;
- a decomposition unit (16) in which formic acid and/or formate is decomposed to carbon monoxide and hydrogen;
- a decomposition unit (18) in which methane is decomposed to carbon and hydrogen; and
- a synthesis unit (17) in which hydrogen and carbon monoxide are converted to methanol.

## Patentansprüche

1. Kohlendioxidnegatives Verfahren zur Herstellung eines Polymerprodukts, umfassend die Schritte:
a) Abscheiden von Kohlendioxid (1, 11.1, 11.2) aus einem Kohlendioxid umfassenden Gas durch direkte Luftabscheidung, wobei in einer ersten Phase das Kohlendioxid umfassende Gas mit Natriumhydroxid in Kontakt gebracht wird, um Kohlendioxid zu absorbieren und Natriumcarbonat zu bilden; und in einer zweiten Phase das in der ersten Phase erhaltene Natriumcarbonat im festen Zustand bereitgestellt und zumindest teilweise thermisch zersetzt wird, wodurch gasförmiges Kohlendioxid freigesetzt und durch die thermische Zersetzung erhaltenes Natriumhydroxid extrahiert wird, und wodurch gasförmiges Kohlendioxid und Natriumhydroxid aus dem in der ersten Phase erhaltenen Natriumcarbonat und/oder aus aus dem in der ersten Phase erhaltenen Natriumcarbonat umgewandeltem Natriumhydrogencarbonat bei einer Temperatur unter 350 °C, vorzugsweise unter 250 °C, z. B. zwischen 50 und 200 °C, bevorzugter zwischen 90 °C und 200 °C, gebildet werden;
b) Umwandeln zumindest eines Teils des abgeschiedenen Kohlendioxids (2, 12.1) in ein Zwischenprodukt, das ein Olefin umfasst, wobei das Olefin Propylen umfasst; vorzugsweise durch
(i) elektrochemische Reduktion (2i, 12.1) des Kohlendioxids in Gegenwart von Wasser; und/oder
(ii) Hydrierung (2ii) des Kohlendioxids mit Wasserstoff zur Bildung von Methanol und anschließendes Umwandeln des Methanols in das Olefin, vorzugsweise durch
▪ eine Methanol-Olefin-Reaktion (MTO); und/oder
▪ eine Methanol-Benzin-Reaktion (MTG);
c) Unterziehen des Propylens des Zwischenprodukts einer Derivatisierungsreaktion (3, 13.1, 13.2), um Acrylnitril als ein Propylenderivat zu erhalten, wobei das Propylen einer Ammonoxidationsreaktion unterzogen wird, um das Acrylnitril zu bilden;
d) weiteres Unterziehen des Acrylnitrils einer Polymerisationsreaktion (4, 14.1, 14.2), um ein Polymerprodukt zu erhalten, wobei Acrylnitril polymerisiert wird, um Polyacrylnitril zu bilden;
und wobei das Polyacrylnitril einer Pyrolysereaktion unterzogen wird, um Kohlenstofffasern zu bilden;
wobei die Kohlenstofffasern, insbesondere in Form von Verbundmaterial und/oder in Form von kohlenstofffaserverstärkten Polymeren, mit einem verarbeitbaren Baumaterial, insbesondere einem verarbeitbaren Mörtel- oder Betonmaterial, gemischt werden, um ein Bauelement für ein Gebäude und/oder eine Infrastruktur, beispielsweise ein Fundament, einen Träger, einen Balken, eine Wand, einen Boden und/oder eine Decke, herzustellen, und wobei mindestens für Schritt a), insbesondere für mindestens die Schritte a) und b), insbesondere für alle Schritte a) - d), Energie, die zur Durchführung des/der Verfahrensschritt(e) erforderlich ist, in Form von erneuerbarer Energie bereitgestellt wird.

2. Verfahren nach Anspruch 1, ferner umfassend einen Schritt des Erzeugens der erneuerbaren Energie, insbesondere mit einer Photovoltaikeinheit und/oder einer Windenergieeinheit und/oder einem Solarwärmekollektor, wobei insbesondere die Energie in einer Anlage erzeugt wird, in der Schritt a), insbesondere die Schritte a) und b) oder alle Schritte, stattfinden.

3. Verfahren nach einem der vorhergehenden Ansprüche, wobei Schritt b) den Schritt des Umwandelns zumindest eines Teils des abgeschiedenen Kohlendioxids (2, 12.1) in ein Zwischenprodukt umfasst, das ein Olefin umfasst, wobei das Olefin Ethylen umfasst, wobei Schritt b) gemäß Option (i) so durchgeführt wird, dass ein Gemisch umfassend Ethylen und mindestens eine Verbindung, die aus Sauerstoff, Ameisensäure, Formiat, Kohlenmonoxid, Wasserstoff, Methan und/oder niederen Alkoholen ausgewählt ist, erhalten wird.

4. Verfahren nach Anspruch 3, wobei die Ameisensäure und/oder das Formiat zu Kohlenmonoxid und Wasserstoff zersetzt wird und/oder das Methan zu Kohlenstoff und Wasserstoff zersetzt wird.

5. Verfahren nach Anspruch 3 oder 4, wobei der Wasserstoff und das Kohlenmonoxid zu Methanol umgesetzt werden, wobei das Kohlenmonoxid direkt aus dem Gemisch umfassend Ethylen und/oder aus der Zersetzung von Ameisensäure und/oder Formiat hervorgeht, und/oder wobei der Wasserstoff direkt aus dem Gemisch umfassend Ethylen und/oder aus der Zersetzung von Ameisensäure und/oder Formiat hervorgeht.

6. Verfahren nach einem der vorhergehenden Ansprüche, wobei Schritt b) gemäß Option (ii) durchgeführt wird und das Kohlendioxid einer katalytischen Hydrierungsreaktion unterzogen wird, um das Methanol zu erhalten, wobei ein Katalysator auf Cu-Basis und/oder ein Katalysator auf In₂O₃-Basis, insbesondere Cu/ZnO/Al₂O₃, verwendet wird, und/oder Schritt b) gemäß Option (ii) durchgeführt wird und das erhaltene Methanol in einer Methanol-Olefin-Reaktion mit einem Zeolithkatalysator, insbesondere einem Siliciumaluminiumphosphat-Zeolithkatalysator, in das Olefin, insbesondere Ethylen und/oder Propylen, umgewandelt wird, und/oder Schritt b) gemäß Option (ii) durchgeführt wird und das erhaltene Methanol in einer Methanol-Benzin-Reaktion mit einem Aluminosilikatkatalysator, z.B. ZSM-5, in das Olefin, insbesondere Ethylen und/oder Propylen, umgewandelt wird.

7. Verfahren nach einem der vorhergehenden Ansprüche, wobei Schritt b) den Schritt des Umwandelns zumindest eines Teils des abgeschiedenen Kohlendioxids (2, 12.1) in ein Zwischenprodukt umfasst, das ein Olefin aufweist, wobei das Olefin Ethylen umfasst, und Unterziehen des Olefins des Zwischenprodukts einer Derivatisierungsreaktion (3, 13.1, 13.2), um ein Glykol zu erhalten, wobei in Schritt d) das erhaltene Glykol, insbesondere Monoethylenglykol, mit Terephthalsäure oder 2,5-Furandicarbonsäure polymerisiert wird, um Polyethylenterephthalat (PET) oder Polyethylenfuranoat (PEF) als das Polymerprodukt zu erhalten, wobei vorzugsweise die 2,5-Furandicarbonsäure aus Biomasse, vorzugsweise aus Fructose, insbesondere über die Zwischenverbindung 5-Hydroxmethylfurfural, erhalten wird.

8. Verfahren nach Anspruch 7, wobei in Schritt d) das Olefin und/oder das Glykol in Polyethylenglykol (PEG), Polypropylen (PP) und/oder Polyethylen (PE) umgewandelt wird.

9. Verfahren nach einem der vorhergehenden Ansprüche, wobei das verarbeitbare Baumaterial vorzugsweise ein Geopolymerbindemittel umfasst.

10. Vorrichtung, insbesondere eine chemische Anlage, zum Durchführen des Verfahrens nach einem der Ansprüche 1 - 9, umfassend:
a) Abscheidungseinheit (1), insbesondere eine Kohlendioxidsorptionseinheit, die zum Abscheiden von Kohlendioxid aus einem Kohlendioxid umfassenden Gas, insbesondere aus Luft oder Abgas, insbesondere durch direkte Luftabscheidung, konfiguriert ist, und wobei in einer ersten Phase das Kohlendioxid umfassende Gas mit Natriumhydroxid, insbesondere einer Natriumhydroxidlösung und/oder festem Natriumhydroxid, in Kontakt gebracht werden kann, um Kohlendioxid zu absorbieren und Natriumcarbonat zu bilden; und in einer zweiten Phase das in der ersten Phase erhaltene Natriumcarbonat im festen Zustand bereitgestellt und zumindest teilweise thermisch zersetzt werden kann, wodurch gasförmiges Kohlendioxid freigesetzt und durch die thermische Zersetzung erhaltenes Natriumhydroxid extrahiert werden kann, insbesondere parallel zur thermischen Zersetzung des Natriumcarbonats, vorzugsweise durch ein Extraktionsmittel;
b) Umwandlungseinheit (2), die zum Umwandeln zumindest eines Teils des mit der Abscheidungseinheit abgeschiedenen Kohlendioxids in ein Zwischenprodukt konfiguriert ist, das ein Olefin, insbesondere Ethylen und/oder Propylen, umfasst; wobei die Umwandlungseinheit vorzugsweise umfasst:
- Reduktionseinheit (2i) zur elektrochemischen Reduktion des Kohlendioxids in Gegenwart von Wasser; und/oder
- Hydrierungseinheit (2ii) zur Hydrierung des Kohlendioxids mit Wasserstoff zur Bildung von Methanol, und anschließendes Umwandeln des Methanols in das Olefin;
c) optional eine Derivatisierungseinheit (3) zum Derivatisieren zumindest eines Teils des Olefins des Zwischenprodukts in ein Olefinderivat;
d) Polymerisationseinheit (4) zum Polymerisieren des Olefins und/oder Olefinderivats, insbesondere mit mindestens einem weiteren Monomer, um ein Polymerprodukt zu erhalten.

11. Vorrichtung nach Anspruch 10, ferner umfassend eine Energieeinheit, die zum Erzeugen von erneuerbarer Energie konfiguriert ist, insbesondere eine Photovoltaikeinheit, eine Solarwärmeeinheit und/oder eine Windenergieeinheit.

12. Vorrichtung nach Anspruch 10 oder 11, ferner umfassend mindestens eine Einheit, ausgewählt aus
- einer Trenneinheit (12.2), in der Nebenprodukte, insbesondere mindestens eine Verbindung, die aus Sauerstoff, Ameisensäure, Formiat, Kohlenmonoxid, Wasserstoff, Methan und/oder niederen Alkoholen ausgewählt ist, von dem Zwischenprodukt, das das Olefin umfasst, getrennt werden können;
- einer Zersetzungseinheit (16), in der Ameisensäure und/oder Formiat zu Kohlenmonoxid und Wasserstoff zersetzt wird;
- einer Zersetzungseinheit (18), in der Methan zu Kohlenstoff und Wasserstoff zersetzt wird; und
- einer Syntheseeinheit (17), in der Wasserstoff und Kohlenmonoxid zu Methanol umgewandelt werden.

## Revendications

1. Procédé à empreinte de dioxyde de carbone négative, pour la préparation d'un produit polymère, comprenant les étapes suivantes:
a) capturer du dioxyde de carbone (1, 11.1, 11.2) à partir d'un gaz comprenant du dioxyde de carbone par capture directe de l'air, dans lequel, dans une première étape, le gaz comprenant du dioxyde de carbone est mis en contact avec de l'hydroxyde de sodium pour absorber le dioxyde de carbone et pour former du carbonate de sodium; et, dans une deuxième étape, le carbonate de sodium obtenu au cours de la première étape est fourni à l'état solide et au moins partiellement décomposé thermiquement, ce qui libère le dioxyde de carbone gazeux et extrait l'hydroxyde de sodium obtenu par la décomposition thermique, et ce qui forme du dioxyde de carbone gazeux et de l'hydroxyde de sodium à partir du carbonate de sodium obtenu au cours de la première étape et/ou à partir d'hydrogénocarbonate de sodium converti à partir du carbonate de sodium obtenu au cours de la première étape à une température inférieure à 350 °C, de préférence inférieure à 250 °C, par exemple entre 50 et 200 °C, plus préférablement entre 90 °C et 200 °C;
b) convertir au moins une partie du dioxyde de carbone capturé (2, 12.1) en un produit intermédiaire comprenant une oléfine, dans lequel l'oléfine comprend du propylène; de préférence par
(i) réduction électrochimique (2i, 12.1) du dioxyde de carbone en présence d'eau; et/ou
(ii) hydrogénation (2ii) du dioxyde de carbone avec de l'hydrogène pour former du méthanol et convertir ensuite le méthanol en oléfine, de préférence par
▪ une réaction de conversion du méthanol en oléfine (MTO); et/ou
▪ une réaction de conversion du méthanol en essence (MTG);
c) soumettre le propylène du produit intermédiaire à une réaction de dérivatisation (3, 13.1, 13.2) pour obtenir de l'acrylonitrile en tant que dérivé de propylène, dans lequel le propylène est soumis à une réaction d'ammoxydation pour former l'acrylonitrile;
d) soumettre en outre l'acrylonitrile à une réaction de polymérisation (4, 14.1, 14.2) pour obtenir un produit polymère, dans lequel l'acrylonitrile est polymérisé pour former du polyacrylonitrile;
et dans lequel le polyacrylonitrile est soumis à une réaction de pyrolyse pour former des fibres de carbone;
dans lequel les fibres de carbone, en particulier sous la forme d'un matériau composite et/ou sous la forme de polymères renforcés par des fibres de carbone, sont mélangées avec un matériau de construction transformable, en particulier un matériau de mortier ou de béton transformable, pour préparer un élément de construction pour un bâtiment et/ou une infrastructure, par exemple une base, un support, une poutre, un mur, un plancher et/ou un plafond, et dans lequel au moins pour l'étape a), en particulier pour au moins les étapes a) et b), en particulier pour toutes les étapes a) à d), l'énergie nécessaire pour réaliser la ou les étapes de procédé est fournie sous la forme d'énergie renouvelable.

2. Procédé selon la revendication 1, comprenant en outre une étape de génération de l'énergie renouvelable, en particulier avec une unité photovoltaïque et/ou une unité d'énergie éolienne et/ou un collecteur thermique solaire, dans lequel, en particulier, l'énergie est générée dans une installation où l'étape a), en particulier les étapes a) et b) ou toutes les étapes, se déroulent.

3. Procédé selon l'une des revendications précédentes, dans lequel l'étape b) comprend l'étape consistant à convertir au moins une partie du dioxyde de carbone capturé (2, 12.1) en un produit intermédiaire comprenant une oléfine, dans lequel l'oléfine comprend de l'éthylène, dans lequel l'étape b) est réalisée selon l'option (i) de telle sorte qu'un mélange comprenant de l'éthylène et au moins un composé choisi parmi l'oxygène, l'acide formique, le formiate, le monoxyde de carbone, l'hydrogène, le méthane et/ou des alcools inférieurs est obtenu.

4. Procédé selon la revendication 3, dans lequel l'acide formique et/ou le formiate est décomposé en monoxyde de carbone et en hydrogène et/ou le méthane est décomposé en carbone et en hydrogène.

5. Procédé selon la revendication 3 ou 4, dans lequel l'hydrogène et le monoxyde de carbone sont convertis en méthanol, dans lequel le monoxyde de carbone provient directement du mélange comprenant de l'éthylène et/ou de la décomposition de l'acide formique et/ou du formiate et/ou dans lequel l'hydrogène provient directement du mélange comprenant de l'éthylène et/ou de la décomposition du méthane.

6. Procédé selon l'une des revendications précédentes, dans lequel l'étape b) est réalisée selon l'option (ii) et le dioxyde de carbone est soumis à une réaction d'hydrogénation catalytique pour obtenir le méthanol, dans lequel un catalyseur à base de Cu et/ou un catalyseur à base d'In₂O₃, en particulier Cu/ZnO/Al₂O₃, est utilisé et/ou l'étape b) est réalisée selon l'option (ii) et le méthanol obtenu est converti en oléfine, en particulier en éthylène et/ou en propylène, au cours d'une réaction de conversion du méthanol en oléfine avec un catalyseur zéolitique, en particulier un catalyseur zéolitique de phosphate de silicium et d'aluminium et/ou l'étape b) est réalisée selon l'option (ii) et le méthanol obtenu est converti en oléfine, en particulier en éthylène et/ou en propylène, au cours d'une réaction de conversion du méthanol en essence avec un catalyseur d'aluminosilicate, par exemple ZSM-5.

7. Procédé selon l'une des revendications précédentes, dans lequel l'étape b) comprend l'étape consistant à convertir au moins une partie du dioxyde de carbone capturé (2, 12.1) en un produit intermédiaire comprenant une oléfine, dans lequel l'oléfine comprend de l'éthylène, et soumettre l'oléfine du produit intermédiaire à une réaction de dérivatisation (3, 13.1, 13.2) pour obtenir un glycol, dans lequel, à l'étape d), le glycol obtenu, en particulier le monoéthylène glycol, est polymérisé avec de l'acide téréphtalique ou de l'acide 2,5-furandicarboxylique pour obtenir du polyéthylène téréphtalate (PET) ou du polyéthylène furandicarboxylat (PEF) en tant que produit polymère, dans lequel, de préférence, l'acide 2,5-furandicarboxylique est obtenu à partir de biomasse, de préférence à partir de fructose, en particulier via le composé intermédiaire 5-hydroxméthylfurfural.

8. Procédé selon la revendication 7, dans lequel, à l'étape d), l'oléfine et/ou le glycol est converti en polyéthylène glycol (PEG), en polypropylène (PP) et/ou en polyéthylène (PE).

9. Procédé selon l'une des revendications précédentes, dans lequel le matériau de construction traitable comprend de préférence un liant géopolymère.

10. Dispositif, en particulier usine chimique, pour réaliser le procédé selon l'une des revendications 1 à 9, comprenant :
a) une unité de capture (1), en particulier une unité de sorption de dioxyde de carbone, qui est configurée pour capturer du dioxyde de carbone à partir d'un gaz comprenant du dioxyde de carbone, en particulier à partir d'air ou de gaz d'échappement, en particulier par capture directe de l'air, et dans lequel, dans une première étape, le gaz comprenant du dioxyde de carbone est configuré de manière à pouvoir être mis en contact avec de l'hydroxyde de sodium, en particulier une solution d'hydroxyde de sodium et/ou de l'hydroxyde de sodium solide, pour absorber le dioxyde de carbone et pour former du carbonate de sodium; et, dans une deuxième étape, le carbonate de sodium obtenu au cours de la première étape est configuré de manière à pouvoir être fourni à l'état solide et au moins partiellement décomposé thermiquement, ce qui libère le dioxyde de carbone gazeux et extrait l'hydroxyde de sodium obtenu par la décomposition thermique, en particulier en parallèle à la décomposition thermique du carbonate de sodium, de préférence par un agent d'extraction;
b) une unité de conversion (2), qui est configurée pour convertir au moins une partie du dioxyde de carbone capturé avec l'unité de capture en un produit intermédiaire comprenant une oléfine, en particulier de l'éthylène et/ou du propylène; dans lequel l'unité de conversion comprend de préférence :
- une unité de réduction (2i) pour la réduction électrochimique du dioxyde de carbone en présence d'eau; et/ou
- une unité d'hydrogénation (2ii) pour l'hydrogénation du dioxyde de carbone avec de l'hydrogène pour former du méthanol et convertir ensuite le méthanol en oléfine;
c) facultativement, une unité de dérivatisation (3) pour dériver au moins une partie de l'oléfine du produit intermédiaire en un dérivé d'oléfine;
d) une unité de polymérisation (4), pour polymériser l'oléfine et/ou le dérivé d'oléfine, en particulier avec au moins un autre monomère, pour obtenir un produit polymère.

11. Dispositif selon la revendication 10, comprenant en outre une unité d'énergie qui est configurée pour produire de l'énergie renouvelable, en particulier une unité photovoltaïque, une unité thermique solaire et/ou une unité d'énergie éolienne.

12. Dispositif selon la revendication 10 ou 11, comprenant en outre au moins une unité choisie parmi
- une unité de séparation (12.2) dans laquelle des sous-produits, en particulier au moins un composé choisi parmi l'oxygène, l'acide formique, le formiate, le monoxyde de carbone, l'hydrogène, le méthane et/ou des alcools inférieurs, sont séparables du produit intermédiaire comprenant l'oléfine;
- une unité de décomposition (16) dans laquelle l'acide formique et/ou le formiate est décomposé en monoxyde de carbone et en hydrogène;
- une unité de décomposition (18) dans laquelle le méthane est décomposé en carbone et en hydrogène; et
- une unité de synthèse (17) dans laquelle l'hydrogène et le monoxyde de carbone sont convertis en méthanol.
